(19) Europäisches Patentamt
European Patent Office
Office européen des brevets

(11) EP 2 792 357 A1

(12) EUROPEAN PATENT APPLICATION
published in accordance with Art. 153(4) EPC

(43) Date of publication:
22.10.2014 Bulletin 2014/43

(21) Application number: 12857962.0

(22) Date of filing: 13.12.2012

(51) Int Cl.:
*A61K 31/4439* (2006.01) *A61P 7/02* (2006.01)
*A61P 9/10* (2006.01) *A61P 13/12* (2006.01)

(86) International application number:
PCT/JP2012/082279

(87) International publication number:
WO 2013/089164 (20.06.2013 Gazette 2013/25)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 14.12.2011 JP 2011273516

(71) Applicant: Daiichi Sankyo Company, Limited
Tokyo 103-8426 (JP)

(72) Inventors:
- KIMURA, Tetsuya
Tokyo 140-8710 (JP)
- KUMAKURA, Tomohiko
Tokyo 140-8710 (JP)
- TACHIBANA, Masaya
Tokyo 140-8710 (JP)
- MATSUMOTO, Chiaki
Tokyo 140-8710 (JP)
- ABE, Kenji
Tokyo 140-8710 (JP)

(74) Representative: Nieuwenhuys, William Francis
Marks & Clerk LLP
90 Long Acre
London
WC2E 9RA (GB)

# (54) THROMBOEMBOLISM PREVENTIVE/THERAPEUTIC AGENT FOR THROMBOEMBOLISM PATIENTS HAVING SEVERE RENAL DYSFUNCTION

(57) It is intended to provide a highly safe, orally administrable preventive and/or therapeutic agent for thromboembolism that is intended for a thromboembolism patient with severe renal impairment. The present invention provides a preventive and/or therapeutic agent for thromboembolism, containing edoxaban, wherein the preventive and/or therapeutic agent is used so that the edoxaban is administered at a dose of 15 mg once a day to an atrial fibrillation patient with severe renal impairment.

EP 2 792 357 A1

## Description

Technical Field

The present invention relates to a preventive and/or therapeutic agent for thromboembolism in a thromboembolism patient with severe renal impairment, containing edoxaban.

Background Art

There has been remarkable development of oral anticoagulant drugs substituting for warfarin in recent years, including newly-discovered direct thrombin inhibitor dabigatran and activated blood coagulation factor X (referred to as FXa herein) inhibitors edoxaban, rivaroxaban, and apixaban.

Edoxaban tosilate hydrate (Patent literature 1 and Patent literature 2) is sold in Japan under the trade name of LIXIANA (registered trademark) tablets for the reduction of occurrence of venous thromboembolism (referred to as VTE herein) in patients undergoing total knee replacement (referred to as TKR herein), total hip replacement (referred to as THR herein), or hip fracture surgery (referred to as HFS herein) as an indication. Edoxaban tosilate hydrate is also under a multinational clinical trial at phase-III for cerebral infarction or systemic embolism attributed to non-valvular atrial fibrillation (referred to as NVAF herein) as an indication (Non-patent literature 1 and Non-patent literature 2).

For the typical dosage and administration of LIXIANA (registered trademark) tablets, 30 mg of edoxaban is orally administered to an adult once a day. The LIXIANA (registered trademark) tablets may cause an elevated serum concentration in patients with renal functional impairment, increasing the risk of bleeding. Thus, in patients with moderate renal impairment (creatinine clearance (referred to as $CL_{CR}$ herein): 30 mL/min or higher and lower than 50 mL/min), the LIXIANA (registered trademark) tablets are administered at an appropriately reduced dose of 15 mg once a day after evaluation of each individual patient for his or her risk of onset of venous thromboembolism and risk of bleeding. In addition, the use of LIXIANA (registered trademark) tablets is contraindicated in patients with severe renal impairment ($CL_{CR}$: lower than 30 mL/min) (Non-patent literature 3). Also, it is known that the dose of a pharmaceutical composition containing edoxaban may be selected on the basis of the reference value of a dose determinant in a patient in need of administration thereof (Patent literature 3).

Edoxaban was subjected to a clinical trial targeting individuals with renal functional impairment, in which edoxaban was studied for its pharmacokinetics in the individuals with renal functional impairment (Non-patent literature 4). In this clinical trial, 15 mg of edoxaban was administered at a single dose to each patient in five groups differing in the severity of renal functional impairment.

Assessment Reports, etc. for LIXIANA (registered trademark) tablets filed for the reduction of occurrence of VTE in patients undergoing TKR, THR, or HFS as an indication or efficacy describe: the applicant's recognition that administration to patients having a $CL_{CR}$ lower than 30 mL/min should be carefully determined in consideration of the risk of VTE and the risk of bleeding, because of few clinical trial results from such patients, and that the dose needs to be reduced even in the case where administration should take place; and the decision by the Pharmaceuticals and Medical Devices Agency and by the Expert Committee of Expert Council that the use of the LIXIANA (registered trademark) tablets should be contraindicated to patients having a $CL_{CR}$ lower than 30 mL/min on the grounds of the unknown safety of LIXIANA (registered trademark) tablets for patients having a $CL_{CR}$ lower than 30 mL/min, the possibility that the benefit of avoiding VTE may be accompanied by the unacceptable risk of bleeding, and an approach other than the administration of the anticoagulant drug being able to reduce the risk of VTE (Non-patent literature 5 to 9).

Dabigatran, rivaroxaban, and apixaban are under clinical trial or have been approved for each thromboembolism as an indication. All of these compounds are under clinical trial with exclusion criteria set for individuals with renal functional impairment or have been approved on the condition that the use of each compound is made at an appropriately reduced dose or with caution, is not recommended, or is contraindicated to individuals with renal functional impairment on the basis of each clinical trial. Edoxaban, rivaroxaban, apixaban, and dabigatran have in common that they are low-molecular compounds acting on a blood coagulation cascade centered on FXa or thrombin, while these compounds are known to largely differ in chemical structure and also in pharmacokinetics such as metabolic pathway, excretion pathway, the rate of protein binding, bioavailability, terminal half-life (referred to as $t_{1/2}$ herein), and/or time to maximum plasma concentration ($t_{max}$) (Non-patent literature 10).

Citation List

Patent literature

Patent literature 1: WO2003/000657
Patent literature 2: WO2003/000680
Patent literature 3: WO2010/071164

Non-patent literature

Non-patent literature 1: Thromb. Haemost. 2010 Sep; 104 (3): 633-41
Non-patent literature 2: Am. Heart J. 2010 Oct; 160 (4): 635-41
Non-patent literature 3: LIXIANA (registered trademark) tablets, package insert, the 2nd revised edition in July 2011
Non-patent literature 4: Homepage of information on ethical pharmaceuticals review (http://www.info.pmda.go.jp/approvalSrch/PharmacySrchInit ?), Pharmaceuticals and Medical Devices Agency, LIXIANA (registered trademark) tablets, The Brief Summary of Application Material, 2.7.6 Summary of Individual Studies, 114-128, published online on July 25, 2011
Non-patent literature 5: Homepage of information on ethical pharmaceuticals review (http://www.info.pmda.go.jp/approvalSrch/PharmacySrchInit ?), Pharmaceuticals and Medical Devices Agency, LIXIANA (registered trademark) tablets, The Brief Summary of Application Material, 2.5 Global Assessment for Clinical Practice 48-76, published online on July 25, 2011
Non-patent literature 6: Homepage of information on ethical pharmaceuticals review (http://www.info.pmda.go.jp/approvalSrch/PharmacySrchInit ?), Pharmaceuticals and Medical Devices Agency, LIXIANA (registered trademark) tablets, The Brief Summary of Application Material, 2.7.2 Clinical Pharmacological Study, 30-32 Section "2.3.2 PK for Renal Functional Impairment in Europe", published online on July 25, 2011
Non-patent literature 7: Assessment Report as of February 9, 2011 for LIXIANA (registered trademark) tablets, 41-43 Section "(2) Validity of Reduced Dose for Renal Functional Impairment Patient and for combined use with P-gp inhibitor", published online on July 25, 2011 Non-patent literature 8: Assessment Report as of February 9, 2011 for LIXIANA (registered trademark) tablets, 66-69 Section "(7)1 Individual with Renal Functional Impairment", published online on July 25, 2011
Non-patent literature 9: Assessment Report as of February 9, 2011 for LIXIANA (registered trademark) tablets, 74-78, published online on July 25, 2011
Non-patent literature 10: Circ. J., 2011, Vol. 75, 1539-1547

Summary of Invention

Technical Problem

There is demand for a highly safe, orally administrable preventive and/or therapeutic agent for thromboembolism in a thromboembolism patient with severe renal impairment. Particularly, atrial fibrillation (referred to as AF herein) patients with severe renal impairment are a population at high risk of thromboembolism and in need of long-term anticoagulant therapy. Thus, there is a demand for the development of a highly safe, orally administrable anticoagulant agent that can be applied to such patients.

Solution to Problem

The present inventors have found that, even for a thromboembolism patient with severe renal impairment, use of edoxaban at a dose of 15 mg once a day can effectively prevent thromboembolism while avoiding the risk of bleeding. The present inventors have also found that, even for a thromboembolism patient with severe renal impairment, edoxaban at a dose of 15 mg once a day can effectively prevent thromboembolism with safety over a long period.
Specifically, the present invention relates to:

[1] a preventive and/or therapeutic agent for thrombosis and/or embolism in a thrombosis and/or embolism patient with severe renal impairment, containing edoxaban, wherein the edoxaban is administered at a dose of 15 mg once a day;
[2] the preventive and/or therapeutic agent according to [1], wherein the patient has a creatinine clearance of 15 mL/min or higher and lower than 30 mL/min;
[3] the preventive and/or therapeutic agent according to [1], wherein the preventive and/or therapeutic agent is administered for at least 15 days continuously and/or intermittently;
[4] the preventive and/or therapeutic agent according to [1], wherein the thrombosis and/or embolism is venous

thromboembolism or thrombosis and/or embolism attributed to atrial fibrillation;

[5] the preventive and/or therapeutic agent according to [4], wherein the venous thromboembolism is venous thromboembolism in a postoperative patient;

[6] the preventive and/or therapeutic agent according to [4], wherein the venous thromboembolism is acute venous thromboembolism;

[7] the preventive and/or therapeutic agent according to [4], wherein the thrombosis and/or embolism attributed to atrial fibrillation is cerebral infarction, systemic embolism, or stroke attributed to atrial fibrillation;

[8] a kit for preventing and/or treating thrombosis and/or embolism in a thrombosis and/or embolism patient with severe renal impairment, comprising a pharmaceutical composition containing edoxaban and an instruction to administer the edoxaban at a dose of 15 mg once a day;

[9] a method for preventing and/or treating thrombosis and/or embolism in a thrombosis and/or embolism patient with severe renal impairment, comprising administering edoxaban at a dose of 15 mg once a day to the patient;

[10] a preventive and/or therapeutic agent for thromboembolism, containing edoxaban, wherein the preventive and/or therapeutic agent is used so that the edoxaban is administered at a dose of 15 mg once a day to an atrial fibrillation patient with severe renal impairment;

[11] the preventive and/or therapeutic agent according to [10], wherein the patient has a creatinine clearance of 15 mL/min or higher and lower than 30 mL/min;

[12] the preventive and/or therapeutic agent according to [10], wherein the preventive and/or therapeutic agent is used for administration for at least 15 days continuously and/or intermittently;

[13] the preventive and/or therapeutic agent according to [10], wherein the thromboembolism is thromboembolism attributed to atrial fibrillation;

[14] the preventive and/or therapeutic agent according to [13], wherein the thromboembolism attributed to atrial fibrillation is cerebral infarction, systemic embolism, or stroke attributed to atrial fibrillation;

[15] the preventive and/or therapeutic agent according to any of [10] to [14], wherein the atrial fibrillation is non-valvular atrial fibrillation; and

[16] a method for preventing and/or treating thromboembolism in an atrial fibrillation patient with severe renal impairment, comprising administering edoxaban at a dose of 15 mg once a day to the patient.

Advantageous Effects of Invention

According to the present invention, thromboembolism can be prevented, suppressed, or treated with edoxaban even in a patient with severe renal impairment.

Description of Embodiments

First, terms used herein will be described.

The term "renal functional impairment" is used herein as a general term for "mild renal impairment", "moderate renal impairment", "severe renal impairment", and "renal failure".

The term "mild renal impairment" used herein is defined in terms of $CL_{CR}$ as a $CL_{CR}$ between 50 mL/min and 80 mL/min inclusive or an individual having a $CL_{CR}$ between 50 mL/min and 80 mL/min inclusive.

The term "moderate renal impairment" used herein is defined in terms of $CL_{CR}$ as a $CL_{CR}$ of 30 mL/min or higher and lower than 50 mL/min or an individual having a $CL_{CR}$ of 30 mL/min or higher and lower than 50 mL/min.

The term "severe renal impairment" used herein is defined in terms of $CL_{CR}$ as a $CL_{CR}$ lower than 30 mL/min or an individual having a $CL_{CR}$ lower than 30 mL/min.

The term "renal failure" used herein refers to more severe renal impairment and is defined in terms of $CL_{CR}$ as a $CL_{CR}$ lower than 15 mL/min or an individual having a $CL_{CR}$ lower than 15 mL/min. The term "renal failure" generally refers to renal functional impairment requiring dialysis. The terms "renal failure", "renal failure patient", and "peritoneal dialysis patient" used herein have the same meaning as each other and may be used interchangeably.

The severity of renal functional impairment is described herein mainly using $CL_{CR}$ values. Those skilled in the art will understand that the severity of renal functional impairment is also indicated by an estimated glomerular filtration rate (eGFR) which can be converted to $CL_{CR}$ and vice versa.

The term "venous thromboembolism" (VTE) is used herein as a general term for deep vein thrombosis (including deep vein thrombosis in postoperative patients and acute deep vein thrombosis) and pulmonary embolism (including pulmonary embolism in postoperative patients and acute pulmonary embolism).

The term "atrial fibrillation" (AF) used herein encompasses, but is not limited to, non-valvular atrial fibrillation (NVAF).

The term "postoperative patient" used herein refers to a patient who has undergone an operation for any reason that is not particularly limited. The "postoperative patient" is not limited to a patient who is hospitalized after an operation

and also encompasses a patient who has been discharged from a hospital after an operation but needs to visit the hospital for the observation of signs of thromboembolism attributed to the operation.

The term "lower limb operation" used herein refers to any operation conducted on a lower limb, and examples thereof include, but are not particularly limited to, lower limb surgery or lower limb orthopedic surgery.

The term "lower limb orthopedic surgery" used herein refers to any lower limb operation conducted in the orthopedic field, and examples thereof include, but are not particularly limited to, TKR, THR, and HFS.

The term "prevention" used herein refers to the prevention of a disease and/or a pathological condition from occurring and also encompasses secondary prevention.

Creatinine clearance ($CL_{CR}$) is calculated herein according to the Cockcroft-Gault equation:

Cockcroft-Gault equation

$$\text{For males: } \{(140 - \text{age}) \times \text{body weight (kg)}\} \div \{72 \times \text{serum creatinine level (mq/dL)}\}$$

$$\text{For females: } [\{(140 - \text{age}) \times \text{body weight (kg)}\} \div \{72 \times \text{serum creatinine level (mg/dL)}\}] \times 0.85$$

Next, the present invention will be described.

$N^1$-(5-chloropyridin-2-yl)-$N^2$-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (I):

[Formula 1]

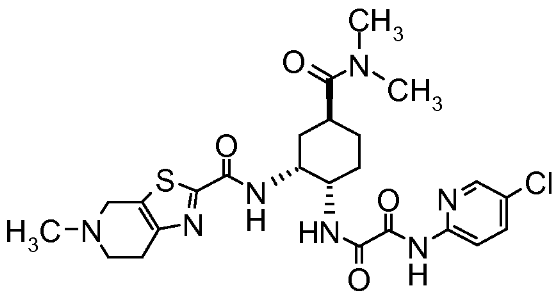

(I)

is called edoxaban (N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide) as International Nonproprietary Name (INN).

LIXIANA (registered trademark) tablets, which comprise edoxaban tosilate monohydrate represented by the following formula (Ia) :

[Formula 2]

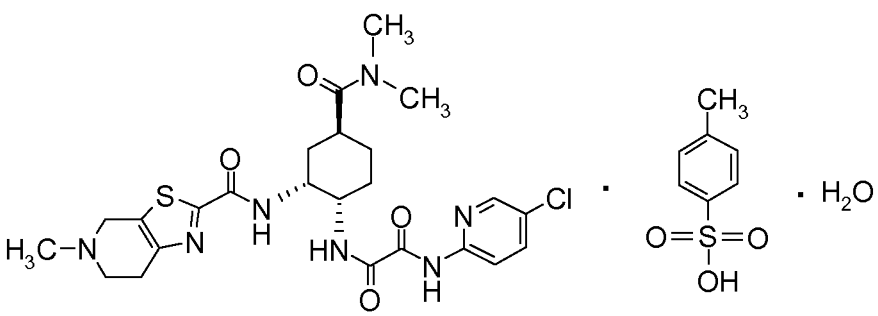

(Ia)

as an active ingredient, are sold in Japan.

Approximately 50% of the edoxaban tosilate monohydrate absorbed in the body is excreted as unchanged drug from the kidney, and the plasma level of edoxaban rises along with a decrease in $CL_{CR}$. In a clinical pharmacological

trial targeting individuals with renal functional impairment, a rise in the area under the plasma (blood) concentration-time curve from 0 to 24 hours after administration (referred to as $AUC_{0-24h}$ herein), a tendency to prolong $t_{1/2}$, a rise in plasma edoxaban concentration 24 hours after administration (referred to as $C_{24h}$ herein), and reduction in renal clearance (referred to as $CL_R$ herein) were observed in the individuals with renal functional impairment compared with healthy adults. $AUC_{0-24h}$ and $C_{24h}$ of individuals with moderate renal impairment increased 1.65 times and 2.52 times respectively, when compared with those of healthy adults. Individuals with severe renal impairment exhibited prolonged $t_{1/2}$ and a rise in $C_{24h}$ compared with the individuals with moderate renal impairment, though no large difference was observed in $AUC_{0-24h}$ or the maximum plasma concentration (referred to as $C_{max}$ herein) therebetween. The degree of the rise in $C_{24h}$ in the individuals with severe renal impairment was approximately 3 times that in healthy adults and approximately 2 times that in the individuals with mild renal impairment (Table 1).

[Table 1]

Table 1 Pharmacokinetic parameters following single-dose administration of Edoxaban at a dose of 15 mg to individuals with renal functional impairment

| | Subject population a) | Healthy adult | Mild renal impairment | Moderate renal impairment | Severe renal impairment | Peritoneal dialysis patient |
|---|---|---|---|---|---|---|
| | The number of test subjects evaluated | 8 | 8 | 8 | 8 | 8 |
| $AUC_{0-24h}$ (ng·h/mL) | Geometric least squares mean | 440 | 569 | 726 | 661 | 770 |
| | Geometric least squares mean ratio to healthy adult(90%CI) | - | 1.29 (1.06, 1.58) | 1.65 (1.35, 2.01) | 1.50 (1.22, 1.84) | 1.75 (1.42, 2.16) |
| $C_{max}$ (ng/mL) | Geometric least squares mean | 88.8 | 104 | 113 | 83.6 | 83.8 |
| | Geometric least squares mean ratio to healthy adult(90%CI) | - | 1.17 (0.850, 1.60) | 1.27 (0.930, 1.74) | 0.941 (0.682, 1.30) | 0.944 (0.679, 1.31) |
| $t_{max}$ (h) | Geometric least squares mean | 1.50 | 1.88 | 1.38 | 1.70 | 2.37 |
| | Geometric least squares mean difference from healthy adult(90%CI) | - | 0.375 (-0.409, 1.16) | -0.125 (-0.909, 0.659) | 0.200 (-0.584, 0.984) | 0.875 (0.090, 1.66) |
| $t_{1/2}$ (h) | Geometric least squares mean | 8.60 | 8.15 | 9.44 | 16.9 | 12.2 |
| | Geometric least squares mean difference from healthy adult(90%CI) | - | -0.440 (-5.26, 4.37) | 0.847 (-3.97, 5.66) | 8.33 (3.51, 13.1) | 3.65 (-1.17, 8.46) |
| $C_{24h}$ (ng/mL) | Geometric mean | 2.34 | 3.44 | 5.90 | 6.88 | 8.24 |
| | CV% | 28.1 | 62.5 | 38.4 | 36.2 | 53.9 |
| $CL_R$ (mL/min) | Geometric mean | 197 | 121 | 67.4 | 32.5 | - |
| | CV% | 16.5 | 37.8 | 37.8 | 49.3 | - |

a: Healthy adult $CL_{CR}$ > 80 mL/min, Mild renal impairment 50mL/min ≤ $CL_{CR}$ ≤ 80 mL/min, Moderate renal impairment 30mL/min ≤ $CL_{CR}$ < 50 mL/min, Severe renal impairment $CL_{CR}$ < 30 mL/min (nondialyzed individual)

Assessment Reports, etc. for LIXIANA (registered trademark) tablets describe: the applicant applied for the possibility of use of the drug being administered at a dose of 15 mg daily for patients having a $CL_{CR}$ lower than 30 mL/min, on the basis of analysis results of Japan TKR phase-II trial targeting the prevention of VTE in patients undergoing TKR, THR, or HFS. The Assessment Reports, etc. also describe the decision by the Pharmaceuticals and Medical Devices Agency and by the Expert Committee of Expert Council that the use of LIXIANA (registered trademark) tablet

should be contraindicated to patients having a $CL_{CR}$ lower than 30 mL/min on the grounds of the unknown safety of LIXIANA (registered trademark) tablets for patients having a $CL_{CR}$ lower than 30 mL/min, the possibility that the benefit of avoiding VTE may be accompanied by the unacceptable risk of bleeding, and the risk of VTE can be reduced by an approach other than the administration of the anticoagulant drug (Non-patent literature 5 to 9).

The package insert of LIXIANA (registered trademark) tablets describes: for a patient undergoing lower limb orthopedic surgery, to use LIXIANA (registered trademark) tablets only during hospitalization after the operation as a rule; the administration period should be determined in consideration of the risks of venous thromboembolism and bleeding in each individual patient; not to continuously administer the drug needlessly after reduction of the risk of venous thromboembolism; and the lack of evaluation of the efficacy and safety of 15-day or longer administration of edoxaban in patients undergoing lower limb orthopedic surgery at the clinical studies in Japan.

When the FXa inhibitor is used as an anticoagulant agent in the prevention and/or treatment of thromboembolism in a postoperative patient, its administration period is short, for example, 5 days after the operation (e.g., after surgery and/or orthopedic surgery; the same holds true for the description below), 1 week after the operation, 10 days after the operation, or 2 weeks after the operation. Alternatively, when the FXa inhibitor is used as an anticoagulant agent in the prevention and/or treatment of acute thromboembolism or in the prevention and/or treatment of thromboembolism in an AF patient, its administration period is long, for example, 5 days or longer, 1 week or longer, 10 days or longer, 2 weeks or longer, 15 days or longer, 1 month or longer, 2 months or longer, 3 months or longer, 4 months or longer, 5 months or longer, or half a year or longer, or 1 year or longer.

Both AF and chronic kidney disease (referred to as CKD herein) are diseases whose prevalence increases with aging. CKD patients have been reported to have particularly high AF prevalence. And, it is said that reduced renal function in AF patients is an independent factor increasing the risk of occurrence of thromboembolism. For these reasons, AF patients with severe renal impairment are a population at high risk of ischemic stroke or systemic thromboembolism and in need of long-term anticoagulant therapy. Meanwhile, patients with severe renal impairment exhibit reduced platelet functions and are thus also a population at high risk of bleeding. At the moment, only warfarin is used as an anticoagulant drug for AF patients with severe renal impairment in Japan and foreign medical practice. Unfortunately, it has been reported that: the dose of warfarin is difficult to adjust due to the difference in the manifestation of pharmacological effect among individuals and the interaction with foods or drugs; and the administration of warfarin tends to increase the incidence of major bleeding depending on the severity of renal functional impairment. Thus, it is important to develop a more manageable, orally administrable anticoagulant drug that is excellent in the balance between the risk of bleeding and preventive effect on the occurrence of thromboembolism in AF patients with severe renal impairment.

No statistical evaluation has been made yet on the safety and/or efficacy of edoxaban administered over a long period (e.g., 15 days or longer) to a patient population in need of treatment of thromboembolism with severe renal impairment.

Preferably, the preventive and/or therapeutic agent of the present invention is administered for 5 days or longer, 1 week or longer, 10 days or longer, 2 weeks or longer, 15 days or longer, 1 month or longer, 2 months or longer, 3 months or longer, 4 months or longer, 5 months or longer, half a year or longer, or 1 year or longer consecutively and/or intermittently. For use in the reduction of occurrence of venous thromboembolism in a postoperative patient, the preventive and/or therapeutic agent of the present invention is preferably administered for 1 to 14 days consecutively and/or intermittently. For use in the prevention and/or treatment of acute venous thromboembolism, the preventive and/or therapeutic agent of the present invention is preferably administered for 5 days or longer, 1 week or longer, 10 days or longer, 2 weeks or longer, 15 days or longer, 1 month or longer, 2 months or longer, 3 months or longer, 4 months or longer, 5 months or longer, half a year or longer, or 1 year or longer consecutively and/or intermittently. For use in the prevention and/or treatment of thromboembolism attributed to atrial fibrillation, for example, cerebral infarction, systemic embolism, or stroke attributed to atrial fibrillation, the preventive and/or therapeutic agent of the present invention is preferably administered for 5 days or longer, 1 week or longer, 10 days or longer, 2 weeks or longer, 15 days or longer, 1 month or longer, 2 months or longer, 3 months or longer, 4 months or longer, 5 months or longer, half a year or longer, or 1 year or longer consecutively and/or intermittently.

The term "severe renal impairment" used herein refers to a $CL_{CR}$ lower than 30 mL/min or an individual having such a $CL_{CR}$ value. Examples of the preferable range of $CL_{CR}$ include 15 mL/min or higher and lower than 30 mL/min.

The term "postoperative patient" used herein preferably refers to a patient 14 days or earlier after an operation.

Examples of the postoperative patient to which the preventive and/or therapeutic agent of the present invention is applied preferably include patients who have undergone a lower limb operation, more preferably patients who have undergone a lower limb operation conducted in the orthopedic field, even more preferably patients who have undergone TKR, THR, or HFS.

The dosage form of the pharmaceutical composition or the preventive and/or therapeutic agent of the present invention can be any orally administrable dosage form and may be a solid or nonsolid preparation, with a solid preparation preferred.

The orally administrable solid preparation is not particularly limited and is preferably tablets (including orally

disintegrating tablets), granules (including fine granules), powders, or capsules. The orally administrable solid preparation can be produced by adopting a well-known method for producing solid preparations.

When the pharmaceutical composition of the present invention is a solid preparation, this solid preparation may comprise a coating agent. The coated solid preparation is not limited to coated solid preparations such as coated tablets and also encompasses various solid preparations comprising coating agents. For example, a solid preparation containing edoxaban or a pharmacologically acceptable salt thereof, or a solvate thereof, wherein coating agents are formulated in a matrix form in the solid preparation is also included in the present invention.

The present invention also relates to a kit for preventing and/or treating thromboembolism in a thromboembolism patient with severe renal impairment, comprising a pharmaceutical composition containing edoxaban and an instruction to administer the edoxaban at a dose of 15 mg once a day. The pharmaceutical composition contained in the kit can have any orally administrable dosage form as described above. Examples of the form of the kit include, but are not particularly limited to, packaged containers comprising a packaged pharmaceutical composition containing edoxaban and an instruction (e.g., package insert) that guides how to take the drug. The instruction may be present as an independent sheet such as a package insert or may be attached to the container containing the pharmaceutical composition containing edoxaban. The accompanying method thereof is not particularly limited.

The present invention further relates to a method for preventing and/or treating thromboembolism in a thromboembolism patient with severe renal impairment, comprising administering 15 mg of edoxaban to the patient once a day. The edoxaban can be administered at a dose of 15 mg to the patient once a day through any route that is not particularly limited and, preferably, is orally administered. For the oral administration, both solid and non-solid preparations can be used, and, preferably a solid preparation, more preferably a tablet is administered. The solid preparation can be in any form by which 15 mg of edoxaban is administered once a day. 15 mg of edoxaban may be contained in one preparation (e.g., in one tablet or one packet) or may be divided into a plurality of preparations (e.g., two or more tablets or two or more packets). Alternatively, the solid preparation is used by splitting. In this form, one dose after the splitting may contain 15 mg of edoxaban. When the solid preparation is a tablet, the tablet may be a tablet to be used by splitting which is designed so that one dose after the splitting contains 15 mg of edoxaban (in this context, the tablet is designed so that its efficacy and safety are appropriately secured for the use of each portion of the split tablet) or may be a tablet containing, for example, 30 mg of edoxaban to be divided into two portions in use (in this context, the tablet is designed so that the efficacy and safety of one dose after the splitting are appropriately secured). Preferable examples of the tablet include tablets each containing 15 mg of edoxaban.

Next, the present invention will be described in detail with reference to Examples. However, the present invention is not intended to be limited to them by any means.

## Examples

(Example 1: Late phase-II trial targeting NVAF patients in Japan)

In Japan, edoxaban 30 mg × 1/day, edoxaban 45 mg × 1/day, or edoxaban 60 mg × 1/day, or warfarin (whose PT-INR was adjusted to 2.0 to 3.0 [1.6 to 2.6 for 70 years or older]) was orally administered for 12 weeks to each of 519 NVAF patients to evaluate the incidence of thromboembolic events and the incidence of bleeding events.

The only thromboembolic event was one cerebral infarction that occurred in one subject in the edoxaban 45 mg group.

Major bleeding occurred in 3 subjects (2.2%) in the edoxaban 45 mg group and 2 subjects (1.5%) in the edoxaban 60 mg group. The incidence of major bleeding or the incidence of major bleeding or clinically relevant non-major bleeding was not statistically significantly different between the warfarin group and each edoxaban group. Likewise, no significant difference was seen in the paired comparison among the edoxaban groups. In addition, a statistically significant dose-response relationship was not observed.

The incidence of bleeding events (major bleeding, clinically relevant non-major bleeding, and minor bleeding in total) was 18.5% (24/130) in the edoxaban 30 mg group, 22.4% (30/134) in the edoxaban 45 mg group, 27.7% (36/130) in the edoxaban 60 mg group, and 20.0% (25/125) in the warfarin group, demonstrating a rise in incidence along with increase in edoxaban dose. The edoxaban 60 mg group exhibited a slightly higher incidence than that of the warfarin group. No statistically significant difference, however, was seen between the warfarin group and each edoxaban group. Likewise, no significant difference was seen in the paired comparison among the edoxaban groups. In addition, statistically significant dose-response relationship was not observed.

A population pharmacokinetic analysis was performed using the plasma edoxaban concentration data of a Japanese late phase-II study in patients with NVAF. As a result, $CL_{CR}$ for clearance was selected as a covariate significantly influencing the pharmacokinetics of edoxaban.

Meanwhile, the relationship of renal function to bleeding, which was the side effect based on the anticoagulant effect of edoxaban, was not clearly confirmed, particularly due to a small number of subjects with moderate renal

impairment ($CL_{CR}$: 30 mL/min or higher and lower than 50 mL/min) and few subjects with severe renal impairment ($CL_{CR}$: lower than 30 mL/min) (Table 2).

[Table 2]

Table 2 Incidence of bleeding events by $CL_{CR}$ in late phase-II trial targeting NVAF patients in Japan

| | Major bleeding or clinically relevant non-major bleeding | | | Bleeding event [a)] | | |
|---|---|---|---|---|---|---|
| | 30 mg | 45 mg | 60 mg | 30 mg | 45 mg | 60 mg |
| $CL_{CR}$ <30 (mL/min) | - | - | 0% (0/1) | - | - | 100.0% (1/1) |
| 30≤ $CL_{CR}$ <50 (mL/min) | 6.7% (1/15) | 9.5% (2/21) | 0% (0/15) | 20.0% (3/15) | 28.6% (6/21) | 26.7% (4/15) |
| 50≤ $CL_{CR}$ ≤80 (mL/min) | 1.4% (1/69) | 4.2% (3/72) | 10.3% (7/68) | 20.3% (14/69) | 26.4% (19/72) | 30.9% (21/68) |
| 80< $CL_{CR}$ (mL/min) | 0% (0/46) | 4.9% (2/41) | 0% (0/46) | 15.2% (7/46) | 12.2% (5/41) | 21.7% (10/46) |

a: major bleeding, clinically relevant non-major bleeding, and minor bleeding in total

The relationship between bleeding events and pharmacokinetic parameters was studied using logistic regression models. As a result, the incidence of bleeding events (major bleeding, clinically relevant non-major bleeding, and minor bleeding in total) was confirmed to correlate with $AUC_{0-24h}$ or $C_{max}$ at steady state or with the plasma edoxaban concentration ($C_{min}$) at trough.

(Example 2: Phase-III trial targeting NVAF patients with severe renal impairment)

<Clinical trial protocol>

After obtaining consent from the subject for participation in the study, his/her $CL_{CR}$ values will be calculated using the Cockcroft-Gault equation at screening to evaluate the renal function of the subject and confirm that he/she is a NVAF patient with severe renal impairment ($CL_{CR}$: 15 mL/min or higher and lower than 30 mL/min (except for hemodialysis patients)) or with normal renal functions or mild renal impairment ($CL_{CR}$: 50 mL/min or higher). The subjects will be enrolled to the study after further examination to confirm he/she fulfills the inclusion/exclusion criteria. Cockcroft-Gault equation

$$\text{For males: } \{(140 - \text{age}) \times \text{body weight (kg)}\} \div \{72 \times \text{serum creatinine level (mg/dL)}\}$$

$$\text{For females: } [\{(140 - \text{age}) \times \text{body weight (kg)}\} \div \{72 \times \text{serum creatinine level (mg/dL)}\}] \times 0.85$$

Subjects with severe renal impairment and NVAF will receive 15 mg of edoxaban once a day for 12 weeks. Subjects with normal renal functions and NVAF or subjects with mild renal impairment and NVAF will receive 30 mg or 60 mg of edoxaban once a day for 12 weeks. The safety and pharmacokinetics of edoxaban in subjects with severe renal impairment and NVAF will be compared with those in subjects with normal renal functions and NVAF or subjects with mild renal impairment and NVAF.

<Selection of study subject>

Inclusion criteria are shown below.

1) NVAF patient with severe renal impairment ($CL_{CR}$: 15 mL/min or higher and lower than 30 mL/min) or with normal renal functions or mild renal impairment ($CL_{CR}$: 50 mL/min or higher)
2) Aged 20 or over
3) Individual who has been confirmed to have atrial fibrillation by electric recording within the past 12 months, can be adapted for anticoagulant therapy, and is to be subjected to anticoagulant therapy during the study period
4) Individual having at least one risk factor for thromboembolism

<Safety endpoint>

1) Incidence of major bleeding or clinically relevant non-major bleeding
2) Incidence of bleeding events (major bleeding, clinically relevant non-major bleeding, or minor bleeding)
3) Incidence of major bleeding
4) Incidence of clinically relevant non-major bleeding
5) Incidence of adverse events
6) Incidence of adverse drug reaction

<Efficacy evaluation>

Cerebral infarction and systemic embolism are defined as thromboembolic events, and the presence or absence of occurrence thereof is examined from the start point of administration of the investigational new drug to the point of visiting a hospital at a follow-up stage (after the completion of a treatment period or on the 2nd week after discontinuation of treatment).

## Claims

**1.** A preventive and/or therapeutic agent for thromboembolism, containing edoxaban, wherein the preventive and/or therapeutic agent is used so that the edoxaban is administered at a dose of 15 mg once a day to an atrial fibrillation patient with severe renal impairment.

**2.** The preventive and/or therapeutic agent according to claim 1, wherein the thromboembolism is cerebral infarction, systemic embolism, or stroke attributed to atrial fibrillation.

**3.** The preventive and/or therapeutic agent according to claim 1 or 2, wherein the patient has a creatinine clearance of 15 mL/min or higher and lower than 30 mL/min.

**4.** The preventive and/or therapeutic agent according to any one of claims 1 to 3, wherein the preventive and/or therapeutic agent is administered for at least 15 days continuously and/or intermittently.

**5.** The preventive and/or therapeutic agent according to any one of claims 1 to 4, wherein the atrial fibrillation is non-valvular atrial fibrillation.

**6.** A kit for preventing and/or treating thromboembolism in an atrial fibrillation patient with severe renal impairment, comprising a pharmaceutical composition containing edoxaban and an instruction to administer the edoxaban at a dose of 15 mg once a day.

**7.** A method for preventing and/or treating thromboembolism in an atrial fibrillation patient with severe renal impairment, comprising administering edoxaban at a dose of 15 mg once a day to the patient.

## INTERNATIONAL SEARCH REPORT

International application No.
PCT/JP2012/082279

A. CLASSIFICATION OF SUBJECT MATTER
*A61K31/4439*(2006.01)i, *A61P7/02*(2006.01)i, *A61P9/10*(2006.01)i, *A61P13/12*(2006.01)n

According to International Patent Classification (IPC) or to both national classification and IPC

B. FIELDS SEARCHED

Minimum documentation searched (classification system followed by classification symbols)
A61K31/4439, A61P7/02, A61P9/10, A61P13/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY/MEDLINE/EMBASE/BIOSIS(STN), JSTPlus/JMEDPlus(JDreamII)

C. DOCUMENTS CONSIDERED TO BE RELEVANT

| Category* | Citation of document, with indication, where appropriate, of the relevant passages | Relevant to claim No. |
|---|---|---|
| Y | WO 2010/071164 A1 (Daiichi Sankyo Co., Ltd.), 24 June 2010 (24.06.2010), claims 1 to 6, 16, 17; example 2; fig. 7 to 9 & EP 2374456 A1 & CN 102325528 A & TW 201026709 A & KR 10-2011-0104491 A | 1-6 |
| Y | Ridout, G. et al., Effect of renal function on edoxaban pharmacokinetics (PK) and on population PK/PK-PD model., Journal of Clinical Pharmacology, 2009.09, Vol. 49, No. 9, pp.1124., Abstract Number: 144. | 1-6 |
| Y | Lixiana (R) Tablets 15mg, Lixiana (R) Tablets 30mg Tenpu Bunsho, 2011.07, http://www.info.pmda.go.jp/downfiles/ph/PDF/430574_3339002F1020_1_02.pdf | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 February, 2013 (05.02.13) | 19 February, 2013 (19.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/082279

C (Continuation). DOCUMENTS CONSIDERED TO BE RELEVANT

| Category* | Citation of document, with indication, where appropriate, of the relevant passages | Relevant to claim No. |
| --- | --- | --- |
| Y | Lixiana Tablets 15mg, Lixiana Tablets 30mg Shinsa Hokokusho, 2011, pages 41 to 43, 66 to 69, http://www.info.pmda.go.jp/shinyaku/P201100072/430574000_22300AMX00547_A100_2.pdf | 1-6 |

Form PCT/ISA/210 (continuation of second sheet) (July 2009)

**INTERNATIONAL SEARCH REPORT**

International application No.
PCT/JP2012/082279

**Box No. II Observations where certain claims were found unsearchable (Continuation of item 2 of first sheet)**

This international search report has not been established in respect of certain claims under Article 17(2)(a) for the following reasons:

1. [x] Claims Nos.: 7
because they relate to subject matter not required to be searched by this Authority, namely:
Claim 7 pertains to "method for treatment of the human body or animal body by surgery or therapy" and thus relates to a subject matter on which this International Searching Authority is not required to carry out an international search under the provisions of PCT Article 17(2)(a)(i) and [PCT Rule 39.1(iv)].

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003000657 A
- WO 2003000680 A
- WO 2010071164 A


### Non-patent literature cited in the description


- *Thromb. Haemost.,* September 2010, vol. 104 (3), 633-41
- *Am. Heart J.,* October 2010, vol. 160 (4), 635-41
- *Circ. J.,* 2011, vol. 75, 1539-1547